# EUROPEAN PATENT SPECIFICATION

(11) **EP 4 525 727 B1**
(45) Date of publication and mention of the grant of the patent: **29.07.2026**
(21) Application number: 23731001.6
(22) Date of filing: 15.05.2023
(51) Int. Cl.: A61B 6/00, A61N 5/10

(54) **VESSEL CONTRAST BASED INFERENCE OF SELECTIVE INTERNAL RADIATION THERAPY DOSIMETRY**
GEFÄSSKONTRASTBASIERTE INFERENZ DER SELEKTIVEN DOSIMETRIE EINER INTERNEN STRAHLENTHERAPIE
INFÉRENCE DE LA DOSIMÉTRIE DE LA RADIOTHÉRAPIE INTERNE SÉLECTIVE BASÉE SUR LE CONTRASTE DES VAISSEAUX

(30) Priority: 16.05.2022 US 202263342487 P
(43) Date of publication of application: 26.03.2025
(73) Proprietor: Varian Medical Systems, Inc., Palo Alto, California 94304 (US)
(72) Inventor: REGENSBURGER, Alois, 91099 Poxdorf (DE)
(74) Representative: Mathisen & Macara LLP
(86) International application number: PCT/US2023/022260
(87) International publication number: WO 2023/224922

(56) References cited:
- US-A1- 2010 074 493
- US-A1- 2012 190 976

## Description

### TECHNICAL FIELD

This disclosure relates to selective internal radiation therapy (SIRT) dosimetry. More specifically, the disclosure relates to a method that provides a dosimetric estimation for partial volumes of the liver or other organs on the basis of a single cone-beam computed tomography (CBCT) angiography, a method of obtaining computed tomography (CT) using a C-arm system, without spectral resolution or dual energy.

### BACKGROUND

For SIRT dosimetry with X-ray visible microbeads, it is necessary to determine the quantity of microbeads present in a tissue volume. This is important especially in the parenchyma (tissue of the liver outside the blood vessels), which is crossed by many fine microarteries. This is challenging because after a SIRT injection blood vessels and parenchyma often show low concentrations of microbeads as soon as the imaging averages over a voxel size / pixel size. In this context, the technical teaching of document US 2012/190976 A1 is acknowledged.

### SUMMARY

The invention is defined in the appended set of claims. The methods and apparatuses described herein are directed to embodiments where X-Ray images of tissue including microbeads are used to estimate the microbead concentration and distribution in tissue of interest and this estimation is used as input to a dosimetric calculation for the treatment of the tissue. Microbeads can be made from MAA (macroaggregated albumin) which are biodegradable macroaggregate of human serum albumin. The microbeads can also be made with a glass or a resin matrix. This process can be used prior to or for treatment of a hypervascularized tumor such as hepatocellular carcinoma (HCC), which is the most common type of primary liver cancer.

From visible concentrations of contrasted microbeads in medium-sized and large blood vessels, the concentration of microbeads in micro-arteries and in the directly adjacent parenchyma or tumor tissue is extrapolated. This extrapolation can be done model-based or via a learned network, which is calibrated or trained on the basis of comparison with other imaging methods or with histological examinations.

The disclosed embodiments have been found to provide a suitable estimation of the concentration of microbeads in micro-arteries and in the directly adjacent parenchyma or tumor tissue without the need for conventional Dual Energy CBCT or subtraction procedure where complicated patient motion compensation and further recording is required.

According to an embodiment, a method includes recording 3D data of an organ of interest after microbeads are administered into a patient to flow to the organ; segmenting arterial vascular structures with visible accumulations of the microbeads around the organ; determining diameters or lumen(s) of the arterial vascular structures; determining or estimating a degree of a filling factor in the arterial vascular structures; generating a microbead density map along the arterial vascular structures; extrapolating the microbead accumulation in parenchyma and/or tumor tissue in the organ; and generating a composite distribution map of the microbeads, the composite distribution map including the arterial vascular structures with visible accumulations of the microbeads and the extrapolated microbead accumulation in the parenchyma and/or tumor tissue in the organ.

According to another embodiment, a system includes an X-ray CT system including: an X-ray source; a detector; a control and processing system to control the X-ray CT system; and a data storage; wherein the X-ray CT system records 3D data of an organ of a patient to which microbeads were administered to flow to the organ, and the processing system: segments arterial vascular structures with visible accumulations of the microbeads around the organ; determines diameters or lumen(s) of the arterial vascular structures; determines or estimates a degree of a filling factor in the arterial vascular structures; generates a microbead density map along the arterial vascular structures; extrapolates the microbead accumulation in parenchyma and/or tumor tissue in the organ; and generates a composite distribution map of the microbeads, the composite map including the arterial vascular structures with visible accumulations of the microbeads and the extrapolated microbead accumulation in the parenchyma and/or tumor tissue in the organ.

According to another embodiment, a non-transitory computer-readable medium includes executable instructions that when executed by a processor cause the processor to perform the steps of: recording 3D data of microbeads in an organ of interest after microbeads are administered into a patient to flow to the organ; segmenting arterial vascular structures with visible accumulations of the microbeads around the organ; determining diameters of the arterial vascular structures; determining or estimating a degree of a filling factor agent in the arterial vascular structures; generating a microbead density map along the arterial vascular structures; extrapolating the microbead accumulation in parenchyma and/or tumor tissue in the organ; and generating a composite distribution map of the microbeads, the composite map including the arterial vascular structures with visible accumulations of the microbeads and the extrapolated microbead accumulation in the parenchyma and/or tumor tissue in the organ.

In an aspect, the method, the system, or the non-transitory computer-readable medium further includes performing a contrasted scan of an arterial structure of the organ of interest prior to the step of the recording 3D data of the organ.

In an aspect, the method, the system, or the non-transitory computer-readable medium further includes simulating a microarterial structure or a microareterial density in an area of a tumor and a healthy portion of the organ.

In an aspect, the method, the system, or the non-transitory computer-readable medium further includes calculating a bead patency or absorption capacity of tumor tissue adjacent to microarteries or a healthy portion of the organ.

In an aspect, the determining or estimating a degree of a filling fraction in the arterial vascular structures is performed based on a Hounsfield Unit value in the arterial vascular structures and a diameter of the arterial vascular structures.

In an aspect, the extrapolating the microbead accumulation in parenchyma and/or tumor tissue in the organ is performed by a learning-based algorithm.

In an aspect, the extrapolating the microbead accumulation in parenchyma and/or tumor tissue in the organ is based on a size of visibly based contrasted blood vessels, a size of a blood supply area, and how many microbeads were transported through a supply vessel to the arterial vascular structures.

In an aspect, the extrapolating the microbead accumulation in parenchyma and/or tumor tissue in the organ is based on dynamic images of microbead flow into the parenchyma and the tumor tissue.

In an aspect, the composite distribution map contains an estimated density of microbeads for each voxel or volume element in the 3D data.

In an aspect, the method, the system, or the non-transitory computer-readable medium further includes predicting on the basis of a size of one of the arterial vascular structures how many microbeads are transported through the arterial vascular structure into the parenchyma and/or the tumor tissue in the organ.

In an aspect, the method, the system, or the non-transitory computer-readable medium further includes detecting clumps of the microbeads and predicting therefrom whether there is a reduction in blood flow due to an embolic effect of the microbeads.

### BRIEF DESCRIPTION OF THE DRAWINGS

The features and advantages of the present disclosure will be more fully disclosed in, or rendered apparent by the following detailed descriptions of example embodiments. The detailed descriptions of the example embodiments are to be considered together with the accompanying drawings wherein like numbers refer to like parts and further wherein:
FIG. 1 represents blood vessels of an organ embedded within or surround by parenchyma or tumor tissue that have been injected with a contrast agent.
FIG. 2 represents medium to large blood vessels of a similar organ as that of FIG. 1 that have been injected with microbeads.
FIG. 3 is an example of an X-ray CT system that can be used to image microbeads and perform a dosimetric estimation according to an embodiment of the present disclosure.
FIG. 4 is a flowchart including steps of a method of imaging microbeads and performing a dosimetric estimation according to an embodiment of the present disclosure.

### DETAILED DESCRIPTION

The description of the exemplary embodiments is intended to be read in connection with the accompanying drawings, which are to be considered part of the entire written description of these disclosures. While the present disclosure is susceptible to various modifications and alternative forms, specific embodiments are shown by way of example in the drawings and will be described in detail herein. The objectives and advantages of the claimed subject matter will become more apparent from the following detailed description of these exemplary embodiments in connection with the accompanying drawings.

It should be understood, however, that the present disclosure is not intended to be limited to the particular forms disclosed. Rather, the present disclosure covers all modifications and alternatives that fall within the scope of these exemplary embodiments.

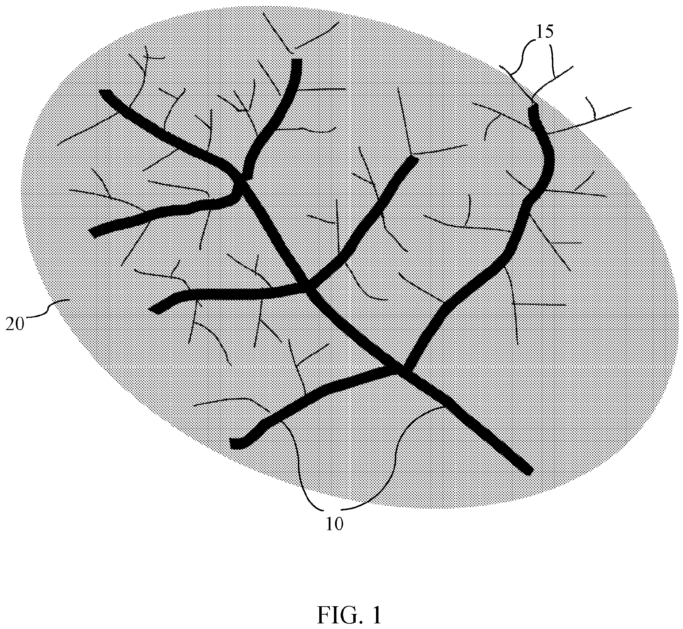

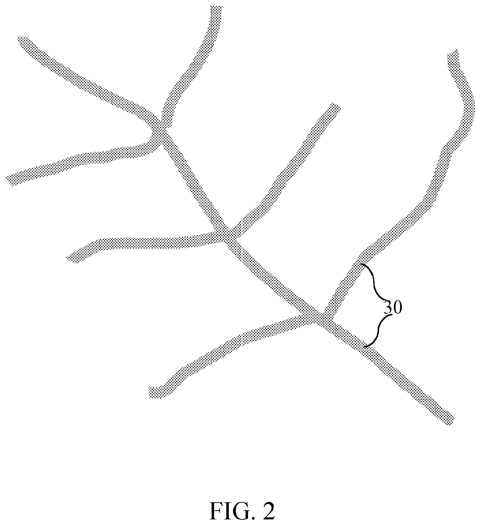

FIGs. 1 and 2 are used to describe the problem. The dark lines in FIG. 1 represent blood vessels of an organ surrounded by parenchyma or tumor tissue 20, represented as a gray oval. The medium to large blood vessels 10 and the microvasculature 15 are filled with a liquid contrast agent such as an iodine solution that has been injected upstream from the tissues. The parenchyma or tumor tissue 20 has a measurable contrast uptake via a body perfused blood volume (DynaPBV body) protocol. It is possible to image small blood vessels and tissue uptake of the liquid contrast agent. This is done by either directly resolving small individual blood vessels or by calculating a bulk/spatially averaged uptake of contrast agent in tissue and small blood vessels.

The lines in FIG. 2 represent medium to large blood vessels of a similar organ as that of FIG. 1 that have been injected with microbeads. When imaged, there is some visibility of the medium to large blood vessels but microbead concentration in microvasculature and parenchyma/tumor tissue is not visible or measureable.

During SIRT, tiny glass or resin beads filled with the radioactive isotope yttrium Y-90 (or possibly non-radioactive) are placed inside the blood vessels that feed a tumor. The injected beads, via the inclusion of highly X-ray visible materials in the small beads, are directly visible in a 3D X-ray image. Typically, individual beads cannot be resolved in imaging, but all larger accumulations of beads, such as in arterial blood vessels of different sizes, are directly visible in the X-ray image. Smaller accumulations or freely distributed beads cannot be resolved without spectral methods such as dual energy or photon counting, and are only diffusely noticeable by a slight increase in the Hounsfield Unit (HU) value. The microbeads can have a diameter of, for example, 15, 20, 30 or 50 microns, or another appropriate size. Due to this larger diameter, the microbeads cannot easily diffuse/penetrate into the parenchyma like the liquid contrast agent. That is, a large portion of the beads typically remains "stuck" in blood vessels of different sizes, or penetrate from these blood vessels only a small distance into the surrounding parenchyma or tumor tissue.

An object of the disclosure is a flow phantom or human or animal organ into which SIRT microbeads are injected and at least a portion of the microbeads is X-ray visible. A C-arm/CBCT scanner or CT or other 3D X-ray imaging system can be used to image the microbeads. A data processing system with associated data storage is used to interpret the data and perform simulations of the microarterial structure or microarterial density in the area of the tumor and in the healthy part of the organ and calculate treatment dosimetry.

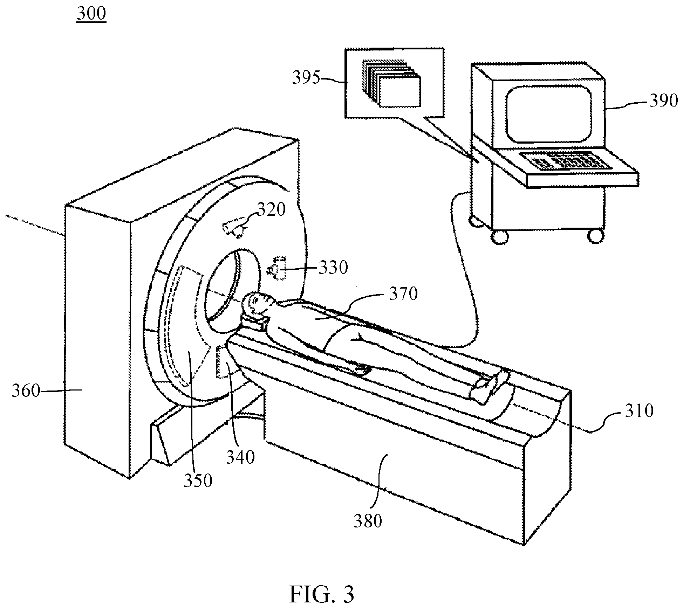

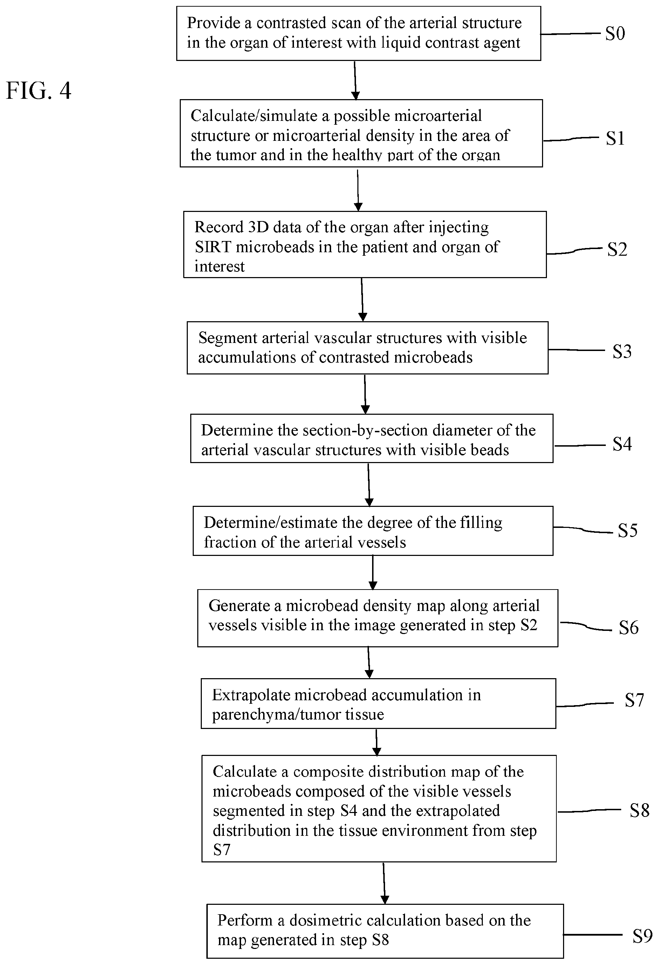

FIG. 3 is an example of an X-ray CT system 300 that can be used to image microbeads and perform a dosimetric estimation according to an embodiment of the present disclosure. The X-ray CT system 300 can include X-ray sources 320 and 330, detectors 340 and 350, a gantry housing 360, a patient 370 on a patient bed 380, a system axis 310, a control and processing system 390, and a data storage 395. The control and processing system 390 can be a single computer or network of computers used to control the X-ray CT system 300, and interact with a technician to perform image capture, image processing, data manipulation, calculations, and map generation in steps of the method described below with respect to FIG. 4.

FIG. 4 is a flowchart including steps of a method of imaging microbeads and performing a dosimetric estimation according to an embodiment of the present disclosure. The disclosed method is meant to be performed prior to and as an input to providing radiation therapy to a patient. Any procedure injecting microbeads or material with different flow/infiltration dynamics than the liquid contrast agent could benefit from this method. For example, such procedures can include transarterial chemoembolization (TACE) with drug eluting beads, and injection of ultrasound contrast bubbles. Using the disclosed method, a technician captures a single CBCT or CT image of a patient after the microbead injection without spectral information, and a complete dosimetry or estimated total distribution of the microbeads is output. No Dual Energy CBCT or subtraction recording is required, but the method could be also used based on Dual Energy or subtraction imaging for even higher sensitivity.

Step S0 can be optionally performed to image or provide a contrasted scan of the arterial structure in the organ of interest with a liquid contrast agent, for example 3D X-ray angiogram, arterial phase of a CT, MRI with contrasted arteries. The optional contrasted scan can be used to identify and segment arterial vessels.

Step S1 can be optionally performed with a computer to calculate/simulate a possible microarterial structure or microarterial density in the area of the tumor and in the healthy part of the organ. Additionally, the method can calculate/assume a bead patency or absorption capacity of the tumor tissue adjacent to the microarteries or healthy parenchyma. For this purpose, the tumor type, blood values, metabolic tests such as an Indocyanine Green Angiography (ICG) test, measured contrast medium dynamics, or other data/examination results can also be used. The microarterial structure includes small and very small arterial vessels that supply the parenchyma and tumor tissue. These vessels are large enough to be at least partially infiltrated by microbeads.

In step S2, by computer, record or provide a 3D data set of the organ or tissue or object of investigation after selective arterial injection of SIRT microbeads is performed on the patient and organ of interest. For example, the 3D data set can be recorded by the imaging device and characterizes the vasculature and visible microbeads included in the vasculature.

In step S3, segment arterial vascular structures with visible accumulations of contrasted microbeads. This can be an automated segmentation or performed by a technician. Alternatively, this step can also be semi-automatic segmentation, i.e. any known combination of computer segmentations with human interaction/correction. For example, that can be threshold-based or via known vascular segmentation algorithms or networks trained for this purpose.

In step S4, typically by computer, determine the section-by-section diameter of the arterial vascular structures with visible beads. This determination can optionally also be carried out via a registration to the 3D data set and/or segmentation of the arterial vessels resulting from step S0.

In step S5, by computer, determine/estimate the degree of the filling fraction of the arterial vessels with visible microbeads from step S3. For example, this is performed around the tissue with the HU value in the portion of the vessel and the diameter of the vessels, whereby partial volume effects are taken into account, i.e. effects of the finite voxel size in the 3D image are included. Optionally, averaging/smoothing/interpolating microbead flow modelling along the arterial vessels can be used.

In step S6, a microbead density map is generated by computer along arterial vessels visible in the image generated in step S2. The microbead density map is the result of steps S5 and S6, meaning it will contain visible vessels, their size, and their filling fraction/filling density with microbeads.

From the visible arteries with microbeads, in step S7 microbead accumulation is now extrapolated to the surrounding parenchyma/tumor tissue surrounded by these arteries and the microarterial vessels contained therein.

In another aspect, a model-based extrapolation of contrast agent density in the microarteries and infiltration can be used. For example, the model or trained function can be calibrated/annotated with histological determinations of the actual concentrations of microbeads.

In another aspect, a learning-based algorithm, which was trained on the basis of paired images of a type capture in step S3 together with "Ground Truth" images such as PET/CT or Dual Energy CT can be used. This means that based on CBCT images, in which only medium-sized and larger arteries can be segmented with microbeads, this learning-based algorithm can extrapolate/estimate what the microbeads density in the microarteries or parenchyma or tumor tissues supplied by the contrasted arteries could be like. An example network architecture used for this task could be a generative adversarial network (GAN).

In another aspect, in the modeling, it can be predicted, in particular on the basis of the size of the visibly contrasted blood vessel and on the basis of the size of the supply area, how many microbeads were transported through the supply vessel into the microarterial structures. Furthermore, on the basis of clumps of the microbeads, it can be predicted whether there was already a "backlog" or "jamming" of the microbeads during the SIRT injection and/or a strong reduction in blood flow due to the embolic effect of the microbeads.

In another aspect, LiverPBV (NeuroPBV transferred to the liver) images can be used to estimate the arterially supplied blood volume within the liver tissue. On the basis of transport models and tissue models, a density of microarterial vessels can be generated from the LiverPBV values. In the case of tumors, in areas with very high PBV values, an increased permeability of the arterial vessel walls can be assumed, which relates to a stronger infiltration of microbeads into the tumor tissue. The effect is roughly comparable to a disorder of the blood-brain barrier caused by brain tumors.

In another aspect, in the context of lung shunt detection, additional/combined with Tc99-loaded biodegradable MAA particles or magnetic resonance detectable contrast agents could also be introduced, via which a relation of the distribution of microbeads in the tissue (parenchyma or tumor tissue) relative to the concentration of microbeads in the supplying arteries can be used.

In another aspect, by measuring the dynamics of inflow, for example using 4D Digital Subtraction Angiography (DSA), but with recording during the SIRT injection instead of a test injection, or by 2D-3D registration of projection images to the vascular tree from a previous imaging, it is possible to estimate how many microbeads are caused by the arterial vessels visible in the dynamic images to flow into the tissue supplied by them.

It is noted that the model-based approaches will work particularly precisely in the healthy/intact liver parenchyma, which can be used here on validated tissue models or models of microarterial supply on the basis of histological examinations and previous imaging on other patients. Especially in healthy liver tissue, it is important to calculate rather small concentrations of SIRT microbeads, as very small dose values are to be detected in this step.

In step S8, generate a composite distribution map of the microbeads composed of the visible arterial vascular structures segmented in step S3 and the extrapolated distribution in the tissue environment from step S7. A composite distribution map could, for example contain, an estimated density of microbeads for each voxel or volume element in the 3D dataset from step S3, which is outside of the vessels with visible microbead accumulations where density was already mapped in step S7. It can be assumed that the density of a contrast agent in the microarteries will be similar to a corresponding density of microbeads. Thus, an infiltration model can be used for the directly adjacent tumor tissue/healthy parenchyma as in the nearest/supplying visible arterial vascular section.

In step S9, perform a dosimetric calculation based on the map generated in step S8. See, for example, E Courtney Henry, et al., Precision Dosimetry in Yttrium-90 Radioembolization through CT Imaging of Radiopaque Microspheres in a Rabbit Liver Model, EJNMMI Physics 9, 21 (2022).

The above-described steps and features can be implemented in any of numerous ways. For example, the embodiments can be implemented using hardware, software or a combination thereof. When implemented in software, the software code can be executed on any suitable processor or collection of processors, whether provided in a single computer or distributed among multiple computers. Such processors can be implemented as integrated circuits, with one or more processors in an integrated circuit component. Though, a processor can be implemented using circuitry in any suitable format.

Additionally, or alternatively, the above-described embodiments can be implemented as a non-transitory computer-readable storage medium embodied thereon a program executable by a processor for performing a method of various embodiments.

Also, the various methods or processes outlined herein can be coded as software that is executable on one or more processors that employ any one of a variety of operating systems or platforms. Additionally, such software can be written using any of a number of suitable programming languages and/or programming or scripting tools, and also can be compiled as executable machine language code or intermediate code that is executed on a framework or virtual machine. Typically, the functionality of the program modules can be combined or distributed as desired in various embodiments.

Also, the embodiments of the disclosure can be embodied as a method, of which an example has been provided. The steps or acts performed as part of the method can be ordered in any suitable way. Accordingly, embodiments can be constructed in which steps are performed in an order different than illustrated, which can include performing some steps concurrently, even though shown as sequential steps in illustrative embodiments.

The foregoing is provided for purposes of illustrating, explaining, and describing embodiments of these disclosures. Modifications and adaptations to these embodiments will be apparent to those skilled in the art and may be made without departing from the scope of these disclosures.

## Claims

1. A method comprising: recording 3D data of an organ of interest after microbeads are administered into a patient to flow to the organ; segmenting arterial vascular structures with visible accumulations of the microbeads around the organ; determining diameters of the arterial vascular structures; determining or estimating a degree of a filling fraction of the arterial vascular structures with the visible accumulations of microbeads; generating a microbead density map along the arterial vascular structures; extrapolating the microbead accumulation in parenchyma and/or tumor tissue in the organ; and generating a composite distribution map of the microbeads, the composite distribution map including the arterial vascular structures with visible accumulations of the microbeads and the extrapolated microbead accumulation in the parenchyma and/or tumor tissue in the organ.

2. The method of claim 1 further comprising performing a contrasted scan of an arterial structure of the organ of interest prior to the step of the recording 3D data of the organ.

3. The method of claim 1 further comprising simulating a microarterial structure or a microareterial density in an area of a tumor and a healthy portion of the organ.

4. The method of claim 1 further comprising calculating a bead patency or absorption capacity of tumor tissue adjacent to microarteries or a healthy portion of the organ.

5. The method of claim 1, wherein the determining or estimating a degree of a filling fraction in the arterial vascular structures is performed based on a Hounsfield Unit value in the arterial vascular structures and a diameter of the arterial vascular structures.

6. A system comprising: an X-ray CT system including: an X-ray source; a detector; a control and processing system to control the X-ray CT system; and a data storage; wherein the X-ray CT system is configured to record 3D data of an organ of a patient to which microbeads were administered to flow to the organ, and the control and processing system is configured to: segment arterial vascular structures with visible accumulations of the microbeads around the organ; determine diameters of the arterial vascular structures; determine or estimate a degree of a filling factor of the arterial vascular structures with the visible accumulations of microbeads; generate a microbead density map along the arterial vascular structures; extrapolate the microbead accumulation in parenchyma and/or tumor tissue in the organ; and generate a composite distribution map of the microbeads, the composite map including the arterial vascular structures with visible accumulations of the microbeads and the extrapolated microbead accumulation in the parenchyma and/or tumor tissue in the organ.

7. The method of claim 1 or the system of claim 6, wherein the determining or estimating a degree of a filling fraction in the arterial vascular structures is performed based on a Hounsfield Unit value in the arterial vascular structures and a diameter of the arterial vascular structures.

8. The method of claim 1 or the system of claim 6, wherein the extrapolating the microbead accumulation in parenchyma and/or tumor tissue in the organ is performed by a learning-based algorithm.

9. The method of claim 1 or the system of claim 6, wherein the extrapolating the microbead accumulation in parenchyma and/or tumor tissue in the organ is based on a size of visibly based contrasted blood vessels, a size of a blood supply area, and how many microbeads were transported through a supply vessel to the arterial vascular structures.

10. The method of claim 1 or the system of claim 6, wherein the extrapolating the microbead accumulation in parenchyma and/or tumor tissue in the organ is based on dynamic images of microbead flow into the parenchyma and the tumor tissue.

11. The method or system of any of claims 1 to 10 further comprising performing a dosimetric calculation based on the composite distribution map.

12. A non-transitory computer-readable medium including executable instructions that when executed by a processor cause the processor to perform the steps of: recording 3D data of microbeads in an organ of interest after microbeads are administered into a patient to flow to the organ; segmenting arterial vascular structures with visible accumulations of the microbeads around the organ; determining diameters of the arterial vascular structures; determining or estimating a degree of a filling factor agent of the arterial vascular structures with the visible accumulations of microbeads; generating a microbead density map along the arterial vascular structures; extrapolating the microbead accumulation in parenchyma and/or tumor tissue in the organ; and generating a composite distribution map of the microbeads, the composite map including the arterial vascular structures with visible accumulations of the microbeads and the extrapolated microbead accumulation in the parenchyma and/or tumor tissue in the organ.

13. The method of claim 1, the system of claim 6, or the non-transitory computer-readable medium of claim 12 wherein the composite distribution map contains an estimated density of microbeads for each voxel or volume element in the 3D data.

14. The method of claim 1, the system of claim 6, or the non-transitory computer-readable medium of claim 12 or 13 including predicting on the basis of a size of one of the arterial vascular structures how many microbeads are transported through the arterial vascular structure into the parenchyma and/or the tumor tissue in the organ.

15. The method of claim 1, the system of claim 6, or the non-transitory computer-readable medium of claim 12, 13, or 14 including detecting clumps of the microbeads and predicting therefrom whether there is a reduction in blood flow due to an embolic effect of the microbeads.

## Patentansprüche

1. Verfahren, umfassend: Aufzeichnen von 3D-Daten eines Organs von Interesse, nachdem Mikrokügelchen in einen Patienten verabreicht wurden, um zu dem Organ zu fließen; Segmentieren arterieller Gefäßstrukturen mit sichtbaren Ansammlungen der Mikrokügelchen um das Organ herum; Bestimmen von Durchmessern der arteriellen Gefäßstrukturen; Bestimmen oder Schätzen eines Grades einer Füllfraktion der arteriellen Gefäßstrukturen mit den sichtbaren Ansammlungen von Mikrokügelchen; Erzeugen einer Mikrokügelchen-Dichtekarte entlang der arteriellen Gefäßstrukturen; Extrapolieren der Mikrokügelchen-Ansammlung in Parenchym und/oder Tumorgewebe in dem Organ; und Erzeugen einer zusammengesetzten Verteilungskarte der Mikrokügelchen, wobei die zusammengesetzte Verteilungskarte die arteriellen Gefäßstrukturen mit sichtbaren Ansammlungen der Mikrokügelchen und die extrapolierte Mikrokügelchen-Ansammlung in dem Parenchym und/oder Tumorgewebe in dem Organ einschließt.

2. Verfahren nach Anspruch 1, ferner umfassend Durchführen eines kontrastierten Scans einer arteriellen Struktur des Organs von Interesse vor dem Schritt des Aufzeichnens von 3D-Daten des Organs.

3. Verfahren nach Anspruch 1, ferner umfassend Simulieren einer mikroarteriellen Struktur oder einer mikroarteriellen Dichte in einem Bereich eines Tumors und einem gesunden Abschnitt des Organs.

4. Verfahren nach Anspruch 1, ferner umfassend Berechnen einer Kügelchendurchgängigkeit oder Absorptionskapazität von Tumorgewebe benachbart zu Mikroarterien oder einem gesunden Abschnitt des Organs.

5. Verfahren nach Anspruch 1, wobei das Bestimmen oder Schätzen eines Grades einer Füllfraktion in den arteriellen Gefäßstrukturen basierend auf einem Hounsfield-Einheitenwert in den arteriellen Gefäßstrukturen und einem Durchmesser der arteriellen Gefäßstrukturen durchgeführt wird.

6. System, umfassend: ein Röntgen-CT-System, einschließlich: eine Röntgenquelle; einen Detektor; ein Steuer- und Verarbeitungssystem zum Steuern des Röntgen-CT-Systems; und einen Datenspeicher; wobei das Röntgen-CT-System konfiguriert ist, um 3D-Daten eines Organs eines Patienten aufzuzeichnen, dem Mikrokügelchen verabreicht wurden, um zu dem Organ zu fließen, und das Steuer- und Verarbeitungssystem konfiguriert ist, um: arterielle Gefäßstrukturen mit sichtbaren Ansammlungen der Mikrokügelchen um das Organ herum zu segmentieren; Durchmesser der arteriellen Gefäßstrukturen zu bestimmen; einen Grad eines Füllfaktors der arteriellen Gefäßstrukturen mit den sichtbaren Ansammlungen von Mikrokügelchen zu bestimmen oder zu schätzen; eine Mikrokügelchen-Dichtekarte entlang der arteriellen Gefäßstrukturen zu erzeugen; die Mikrokügelchen-Ansammlung in Parenchym und/oder Tumorgewebe in dem Organ zu extrapolieren; und eine zusammengesetzte Verteilungskarte der Mikrokügelchen zu erzeugen, wobei die zusammengesetzte Karte die arteriellen Gefäßstrukturen mit sichtbaren Ansammlungen der Mikrokügelchen und die extrapolierte Mikrokügelchen-Ansammlung in dem Parenchym und/oder Tumorgewebe in dem Organ einschließt.

7. Verfahren nach Anspruch 1 oder System nach Anspruch 6, wobei das Bestimmen oder Schätzen eines Grades einer Füllfraktion in den arteriellen Gefäßstrukturen basierend auf einem Hounsfield-Einheitenwert in den arteriellen Gefäßstrukturen und einem Durchmesser der arteriellen Gefäßstrukturen durchgeführt wird.

8. Verfahren nach Anspruch 1 oder System nach Anspruch 6, wobei das Extrapolieren der Mikrokügelchen-Ansammlung in Parenchym und/oder Tumorgewebe in dem Organ durch einen lernbasierten Algorithmus durchgeführt wird.

9. Verfahren nach Anspruch 1 oder System nach Anspruch 6, wobei das Extrapolieren der Mikrokügelchen-Ansammlung in Parenchym und/oder Tumorgewebe in dem Organ auf einer Größe von sichtbar basierten kontrastierten Blutgefäßen, einer Größe eines Blutversorgungsbereichs und wie viele Mikrokügelchen durch ein Versorgungsgefäß zu den arteriellen Gefäßstrukturen transportiert wurden, basiert.

10. Verfahren nach Anspruch 1 oder System nach Anspruch 6, wobei das Extrapolieren der Mikrokügelchen-Ansammlung in Parenchym und/oder Tumorgewebe in dem Organ auf dynamischen Bildern von Mikrokügelchenfluss in das Parenchym und das Tumorgewebe basiert.

11. Verfahren oder System nach einem der Ansprüche 1 bis 10, ferner umfassend Durchführen einer dosimetrischen Berechnung basierend auf der zusammengesetzten Verteilungskarte.

12. Nichtflüchtiges computerlesbares Medium, einschließlich ausführbare Anweisungen, die, wenn sie von einem Prozessor ausgeführt werden, den Prozessor veranlassen, die folgenden Schritte durchzuführen: Aufzeichnen von 3D-Daten von Mikrokügelchen in einem Organ von Interesse, nachdem Mikrokügelchen in einen Patienten verabreicht wurden, um zu dem Organ zu fließen; Segmentieren von arteriellen Gefäßstrukturen mit sichtbaren Ansammlungen der Mikrokügelchen um das Organ herum; Bestimmen von Durchmessern der arteriellen Gefäßstrukturen; Bestimmen oder Schätzen eines Grades eines Füllfaktormittels der arteriellen Gefäßstrukturen mit den sichtbaren Ansammlungen von Mikrokügelchen; Erzeugen einer Mikrokügelchen-Dichtekarte entlang der arteriellen Gefäßstrukturen; Extrapolieren der Mikrokügelchen-Ansammlung in Parenchym und/oder Tumorgewebe in dem Organ; und Erzeugen einer zusammengesetzten Verteilungskarte der Mikrokügelchen, wobei die zusammengesetzte Karte die arteriellen Gefäßstrukturen mit sichtbaren Ansammlungen der Mikrokügelchen und die extrapolierte Mikrokügelchen-Ansammlung in dem Parenchym und/oder Tumorgewebe in dem Organ einschließt.

13. Verfahren nach Anspruch 1, System nach Anspruch 6 oder nichtflüchtiges computerlesbares Medium nach Anspruch 12, wobei die zusammengesetzte Verteilungskarte eine geschätzte Dichte von Mikrokügelchen für jedes Voxel oder Volumenelement in den 3D-Daten enthält.

14. Verfahren nach Anspruch 1, System nach Anspruch 6 oder nichtflüchtiges computerlesbares Medium nach Anspruch 12 oder 13, einschließlich Vorhersagen, wie viele Mikrokügelchen durch die arterielle Gefäßstruktur in das Parenchym und/oder das Tumorgewebe in dem Organ transportiert werden, basierend auf einer Größe von einer der arteriellen Gefäßstrukturen.

15. Verfahren nach Anspruch 1, System nach Anspruch 6 oder nichtflüchtiges computerlesbares Medium nach Anspruch 12, 13 oder 14, einschließlich Detektieren von Klumpen der Mikrokügelchen und Vorhersagen daraus, ob es eine Verringerung des Blutflusses aufgrund eines embolischen Effekts der Mikrokügelchen gibt.

## Revendications

1. Procédé comprenant : l'enregistrement de données 3D d'un organe d'intérêt après que des microbilles aient été administrées à un patient pour s'écouler vers l'organe ; la segmentation de structures vasculaires artérielles présentant des accumulations visibles des microbilles autour de l'organe ; la détermination de diamètres des structures vasculaires artérielles ; la détermination ou l'estimation d'un degré d'une fraction de remplissage des structures vasculaires artérielles présentant les accumulations visibles de microbilles ; la génération d'une carte de densité de microbilles le long des structures vasculaires artérielles ; l'extrapolation de l'accumulation de microbilles dans le parenchyme et/ou le tissu tumoral dans l'organe ; et la génération d'une carte de distribution composite des microbilles, la carte de distribution composite comportant les structures vasculaires artérielles présentant des accumulations visibles des microbilles et l'accumulation de microbilles extrapolée dans le parenchyme et/ou le tissu tumoral dans l'organe.

2. Procédé selon la revendication 1, comprenant en outre la réalisation d'un balayage contrasté d'une structure artérielle de l'organe d'intérêt avant l'étape d'enregistrement de données 3D de l'organe.

3. Procédé selon la revendication 1, comprenant en outre la simulation d'une structure microartérielle ou d'une densité microartérielle dans une zone d'une tumeur et une partie saine de l'organe.

4. Procédé selon la revendication 1, comprenant en outre le calcul d'une perméabilité ou d'une capacité d'absorption de billes du tissu tumoral adjacent aux microartères ou à une partie saine de l'organe.

5. Procédé selon la revendication 1, dans lequel la détermination ou l'estimation d'un degré d'une fraction de remplissage dans les structures vasculaires artérielles est réalisée sur la base d'une valeur en unités Hounsfield dans les structures vasculaires artérielles et d'un diamètre des structures vasculaires artérielles.

6. Système comprenant : un système de tomodensitométrie à rayons X comportant : une source de rayons X ; un détecteur ; un système de commande et de traitement pour commander le système de tomodensitométrie à rayons X ; et un système de stockage de données ; dans lequel le système de tomodensitométrie à rayons X est configuré pour enregistrer des données 3D d'un organe d'un patient auquel des microbilles ont été administrées pour s'écouler vers l'organe, et le système de commande et de traitement est configuré pour : segmenter les structures vasculaires artérielles présentant des accumulations visibles des microbilles autour de l'organe ; déterminer les diamètres des structures vasculaires artérielles ; déterminer ou estimer un degré d'une fraction de remplissage des structures vasculaires artérielles présentant les accumulations visibles de microbilles ; générer une carte de densité de microbilles le long des structures vasculaires artérielles ; extrapoler l'accumulation de microbilles dans le parenchyme et/ou le tissu tumoral dans l'organe ; et générer une carte de distribution composite des microbilles, la carte composite comportant les structures vasculaires artérielles présentant des accumulations visibles des microbilles et l'accumulation de microbilles extrapolée dans le parenchyme et/ou le tissu tumoral dans l'organe.

7. Procédé selon la revendication 1 ou système selon la revendication 6, dans lequel la détermination ou l'estimation d'un degré d'une fraction de remplissage dans les structures vasculaires artérielles est réalisée sur la base d'une valeur en unités Hounsfield dans les structures vasculaires artérielles et d'un diamètre des structures vasculaires artérielles.

8. Procédé selon la revendication 1 ou système selon la revendication 6, dans lequel l'extrapolation de l'accumulation de microbilles dans le parenchyme et/ou le tissu tumoral dans l'organe est réalisée par un algorithme basé sur l'apprentissage.

9. Procédé selon la revendication 1 ou système selon la revendication 6, dans lequel l'extrapolation de l'accumulation de microbilles dans le parenchyme et/ou le tissu tumoral dans l'organe est basée sur une taille de vaisseaux sanguins contrastés de manière visible, une taille d'une zone d'approvisionnement en sang et le nombre de microbilles transportées par un vaisseau d'approvisionnement vers les structures vasculaires artérielles.

10. Procédé selon la revendication 1 ou système selon la revendication 6, dans lequel l'extrapolation de l'accumulation de microbilles dans le parenchyme et/ou le tissu tumoral dans l'organe est basée sur des images dynamiques du flux de microbilles dans le parenchyme et le tissu tumoral.

11. Procédé ou système selon l'une quelconque des revendications 1 à 10 comprenant en outre la réalisation d'un calcul dosimétrique sur la base de la carte de distribution composite.

12. Support non transitoire lisible par ordinateur comportant des instructions exécutables qui, lorsqu'elles sont exécutées par un processeur, amènent le processeur à réaliser les étapes suivantes : l'enregistrement de données 3D de microbilles dans un organe d'intérêt après que des microbilles aient été administrées à un patient pour s'écouler vers l'organe ; la segmentation de structures vasculaires artérielles présentant des accumulations visibles des microbilles autour de l'organe ; la détermination de diamètres des structures vasculaires artérielles ; la détermination ou l'estimation d'un degré d'un agent fraction de remplissage des structures vasculaires artérielles présentant les accumulations visibles de microbilles ; la génération d'une carte de densité de microbilles le long des structures vasculaires artérielles ; l'extrapolation de l'accumulation de microbilles dans le parenchyme et/ou le tissu tumoral dans l'organe ; et la génération d'une carte de distribution composite des microbilles, la carte de distribution composite comportant les structures vasculaires artérielles présentant des accumulations visibles des microbilles et l'accumulation de microbilles extrapolée dans le parenchyme et/ou le tissu tumoral dans l'organe.

13. Procédé selon la revendication 1, système selon la revendication 6 ou support non transitoire lisible par ordinateur selon la revendication 12, dans lequel la carte de distribution composite contient une densité estimée de microbilles pour chaque voxel ou élément de volume dans les données 3D.

14. Procédé selon la revendication 1, système selon la revendication 6 ou support non transitoire lisible par ordinateur selon la revendication 12 ou 13, comportant la prédiction, sur la base d'une taille de l'une des structures vasculaires artérielles, du nombre de microbilles transportées par la structure vasculaire artérielle dans le parenchyme et/ou le tissu tumoral dans l'organe.

15. Procédé selon la revendication 1, système selon la revendication 6 ou support non transitoire lisible par ordinateur selon la revendication 12, 13 ou 14, comportant la détection d'amas des microbilles et le fait de prédire, à partir de celle-ci, s'il y a une réduction du flux sanguin due à un effet embolique des microbilles.
